# FASCICULE DE BREVET EUROPEEN

(11) **EP 3 469 254 B1**
(45) Date de publication et mention de la délivrance du brevet: **26.02.2020**
(21) Numéro de dépôt: 16809494.4
(22) Date de dépôt: 15.11.2016
(51) Int. Cl.: F21V 14/02, F21V 21/28, F21W 131/205, F21Y 115/10, F21Y 115/30, F21V 21/40

(54) **DISPOSITIF D'ECLAIRAGE MEDICAL AVEC UN SYSTEME D'AIDE AU BON POSITIONNEMENT**
OPERATIONSLEUCHTE MIT EINEM POSITIONIERUNGSHILFSYSTEM
MEDICAL LIGHTING DEVICE WITH A POSITIONING AID SYSTEM

(30) Priorité: 08.06.2016 FR 1655230
(43) Date de publication de la demande: 17.04.2019
(73) Titulaire: Maquet SAS, 45160 Ardon (FR)
(72) Inventeur: RAVALITERA, Pierre, 45160 Ardon (FR); JEUDI, Thomas, 45160 Ardon (FR)
(74) Mandataire: Prugneau, Philippe
(86) Numéro de dépôt international: PCT/FR2016/052952
(87) Numéro de publication internationale: WO 2017/212126

(56) Documents cités:
- WO-A1-2007/110895
- US-A- 4 639 838
- US-A- 5 068 767
- US-A1- 2003 014 834
- US-A1- 2009 318 772

## Description

### Domaine technique

L'invention concerne de façon générale un dispositif d'éclairage médical d'un champ opératoire comprenant une coupole d'éclairage à éclairage axial qui est destinée à être montée déplaçable au-dessus du champ opératoire de façon à pouvoir être approchée ou éloignée manuellement du champ opératoire, et un système d'aide au réglage d'une bonne position d'éclairage de la coupole par rapport au champ opératoire de telle manière à positionner la coupole à une distance prédéterminée du champ opératoire.

### Technique antérieure

De manière connue, un dispositif d'éclairage utilisé dans un bloc opératoire pour l'éclairage axial d'un champ opératoire comprend une coupole d'éclairage accrochée à un bras de suspension de sorte à être montée déplaçable au dessus du champ opératoire. La position de la coupole est modifiable manuellement par l'équipe médicale du bloc opératoire. Pour manœuvrer la coupole afin de modifier sa position, celle-ci est pourvue d'une poignée permettant de la déplacer et de l'orienter dans l'espace au dessus du champ opératoire.

Il est actuellement de pratique courante de fournir des poignées stériles équipé d'une source de lumière générant un faisceau de lumière laser formant une tache sur le champ opératoire, la tache servant de pointeur lumineux pour aider à orienter et aligner l'appareil d'éclairage au dessus d'un champ opératoire.

On connait aussi du document EP 2 760 257 un dispositif d'éclairage médical dans lequel les sources lumineuses sont contrôlées par une unité de contrôle / commande, cette dernière comprenant aussi un pointeur lumineux générant une tache lumineuse spécifique sur la table opératoire. Dans ce dispositif, il est possible d'orienter automatiquement les sources lumineuses vers cette tache lumineuse, à partir d'une mesure de distance par l'unité de contrôle / commande entre les sources lumineuses et la tache lumineuse sur la table opératoire.

Dans les dispositifs décrits ci-dessus, le pointeur lumineux est donc utilisé principalement pour indiquer le centre d'une zone d'éclairement ou pour mesurer une distance entre l'éclairage et une zone d'éclairage d'intérêt.

Mais dans un bloc opératoire, lors du positionnement manuel d'une coupole d'éclairage par rapport au champ opératoire par le personnel médical, ce dernier ne sait pas vraiment si la coupole est bien placée par rapport au champ opératoire, par exemple il ne sait pas si le faisceau lumineux est bien centré ou orientée angulairement correctement sur la blessure d'un patient, ou encore si la coupole est placée à une hauteur correcte, c'est-à-dire ni trop proche, ni trop éloignée du champ opératoire, de sorte que dans cette position dite correcte l'opérateur puisse profiter des performances optiques optimales de la coupole d'éclairage.

Le document US 2003/0014834 montre un dispositif d'éclairage d'un champ opératoire selon le préambule de la revendication 1.

### Exposé de l'invention

Le but de l'invention est de remédier à ces inconvénients en proposant un dispositif d'éclairage médical permettant de palier visuellement à ces interrogations.

Plus particulièrement, l'invention a pour objet un dispositif d'éclairage médical d'un champ opératoire qui peut comprendre une coupole d'éclairage à éclairage axial qui est destinée à être montée déplaçable au-dessus du champ opératoire de façon à pouvoir être approchée ou éloignée manuellement du champ opératoire, et un système d'aide au réglage d'une bonne position d'éclairage de la coupole par rapport au champ opératoire de telle manière à positionner la coupole à une distance prédéterminée du champ opératoire, caractérisé en ce que le système d'aide au réglage peut comprendre au moins une première source de lumière pour générer un premier faisceau de lumière et une seconde source de lumière pour générer un second faisceau de lumière, en ce que les sources de lumière sont disposées sur la coupole en décalage angulaire de telle manière que les premier et second faisceaux de lumière convergent en un point sur le champ opératoire quand la distance prédéterminée est atteinte et qu'en dehors de cette distance les deux faisceaux de lumière forment deux taches d'éclairement qui sont disjointes.

Le dispositif d'éclairage médical selon l'invention peut encore présenter les particularités suivantes :
- il peut comprendre en outre une troisième source de lumière pour générer un troisième faisceau de lumière, en ce que les première, seconde et troisième sources de lumière sont disposées sur la coupole en décalage angulaire de telle manière que les premier, second et troisième faisceaux de lumière convergent au point sur le champ opératoire quand la distance prédéterminée est atteinte et qu'en dehors de la distance les trois faisceaux de lumière forment trois taches d'éclairement qui sont disjointes ;
- chaque faisceau de lumière peut projeter sur le champ opératoire une tache d'éclairement en forme de cercle plein, et de sorte que les cercles pleins sont confondus sur le point lorsque la coupole est à la distance prédéterminée du champ opératoire ;
- chaque faisceau de lumière peut projeter sur le champ opératoire une tache d'éclairement en forme de cercle évidé, et de sorte que les cercles évidés sont confondus sur le point lorsque la coupole est à la distance prédéterminée du champ opératoire ;
- chaque faisceau de lumière peut projeter sur le champ opératoire une tache d'éclairement en forme de pointe de flèche, de sorte que les pointes de flèche sont orientées vers le point lorsque la coupole est à une distance supérieure à la distance prédéterminée du champ opératoire, de sorte que les pointes de flèche converge vers le point lorsque la coupole est à la distance prédéterminée du champ opératoire et de sorte que les pointes de flèches s'éloignent du point lorsque la coupole est à une distance inférieure à la distance prédéterminée du champ opératoire ;
- les sources de lumière du système d'aide au réglage peuvent être des sources de lumière laser ;
- les sources de lumière du système d'aide au réglage peuvent être des LEDs.

Avec cet agencement selon l'invention, on obtient un dispositif d'éclairage médical avec une coupole à éclairage axiale avec lequel il est possible de positionner manuellement la coupole de manière optimale au dessus du champ opératoire, c'est-à-dire de sorte à bénéficier des performances optiques optimales du dispositif d'éclairage pour une distance prédéterminée de la coupole par rapport au champ opératoire. Un opérateur appartenant au personnel médical, peut avant l'opération ou au cours de l'opération, déplacer la coupole pour illuminer une zone d'intérêt du champ opératoire et positionner ou repositionner la coupole à la distance prédéterminée du champ opératoire, simplement en s'aidant visuellement des sources de lumière générant des faisceaux de lumière qui projettent sur le champ opératoire des taches d'éclairement de sorte que lorsque les faisceaux se croisent sur un même point, les taches d'éclairement convergent sur ce point à la certaine distance prédéterminée.

Selon l'invention, la forme des taches d'éclairement projetées peut aider visuellement l'opérateur à déterminer si la coupole est trop proche, trop éloignée de la zone d'intérêt du champ opératoire, ou à régler la bonne position, c'est-à-dire lorsque la distance prédéterminée par rapport au champ opératoire est atteinte.

Selon l'invention, en multipliant les faisceaux de lumière le système d'aide visuel au positionnement reste fonctionnel même si l'opérateur cache par inadvertance l'un des faisceaux de lumière, par exemple en manipulant la coupole pour la déplacer.

### Présentation sommaire des dessins

La présente invention sera mieux comprise et d'autres avantages apparaîtront à la lecture de la description qui suit et des dessins annexés dans lesquels :

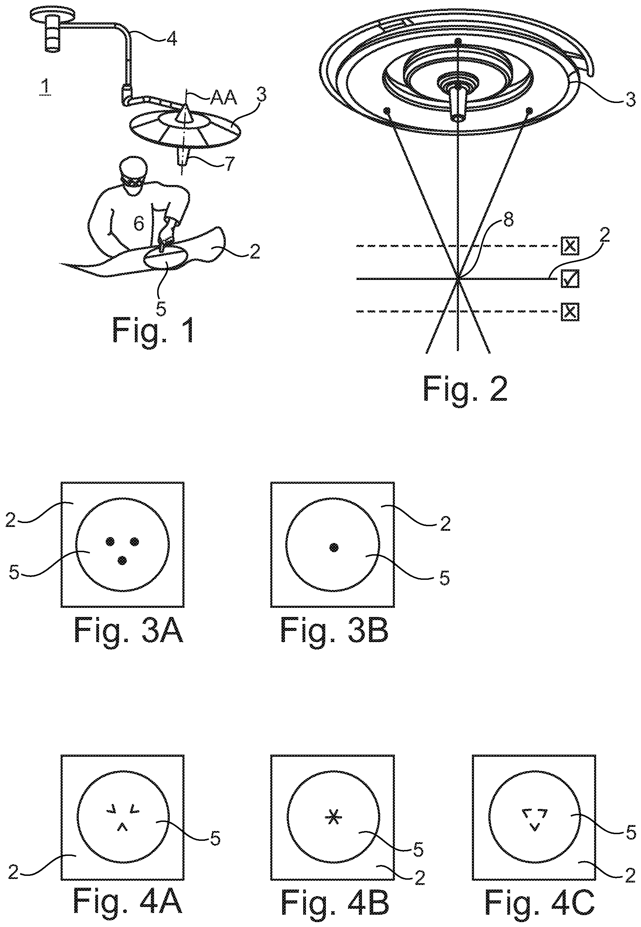

- la figure 1 est une représentation schématique d'un appareil d'éclairage médical selon l'invention avec une coupole d'éclairage qui est montée déplaçable par un opérateur au dessus d'un champ opératoire ;
- la figure 2 est une vue en perspective d'une coupole d'éclairage selon l'invention avec un système d'aide au réglage d'une bonne position d'éclairage de la coupole par rapport au champ opératoire comprenant trois sources de lumière générant chacune un faisceau de lumière en direction du champ opératoire ;
- la figure 3A est une illustration schématique d'une partie du champ opératoire en cas de mauvais positionnement de la coupole d'éclairage par rapport au champ opératoire, la coupole étant équipée de trois sources de lumière projetant chacune sur le champ opératoire une tache d'éclairement en forme de cercle plein selon l'invention ;
- la figure 3B est une illustration schématique d'une partie du champ opératoire en cas de bon positionnement de la coupole d'éclairage par rapport au champ opératoire, la coupole étant équipée de trois sources de lumière projetant chacune sur le champ opératoire une tache d'éclairement en forme de cercle plein selon l'invention ;
- les figures 4A et 4C sont des illustrations schématiques d'une partie du champ opératoire en cas de mauvais positionnement de la coupole d'éclairage par rapport au champ opératoire, la coupole étant équipée de trois sources de lumière projetant chacune sur le champ opératoire une tache d'éclairement en forme de pointe de flèche selon l'invention, la coupole étant respectivement en position trop éloignée du champ opératoire et en position trop proche du champ opératoire ;
- la figure 4B une illustration schématique d'une partie du champ opératoire en cas de bon positionnement de la coupole d'éclairage par rapport au champ opératoire, la coupole étant équipée de trois sources de lumière projetant chacune sur le champ opératoire une tache d'éclairement en forme de pointe de flèche selon l'invention.

### Description d'un mode de réalisation

La figure 1 illustre une partie d'un dispositif d'éclairage 1 médical utilisé dans un bloc opératoire pour éclairer un champ opératoire 2, avec une coupole 3 d'éclairage éclairant le champ opératoire 2 selon l'axe AA, la coupole 3 étant montée sur un bras de suspension 4 articulé, de sorte à pouvoir être déplacée au dessus du champ opératoire 2 en la rapprochant ou l'éloignant manuellement du champ opératoire 2. Ainsi, la coupole 3 est ajustable par rapport au champ opératoire 2 pour éclairer une zone d'intérêt 5 de façon appropriée. Un opérateur 6 appartenant au personnel médical, tel qu'un chirurgien, peut manœuvrer la coupole 3, par exemple à l'aide d'une poignée 7 de préhension agencée ici au centre de la coupole 3, pour la déplacer et l'orienter par rapport au champ opératoire 2.

Le dispositif d'éclairage 1 selon l'invention est équipé d'un système de réglage d'une bonne position d'éclairage de la coupole 3 par rapport au champ opératoire 2 de manière à indiquer à l'opérateur 6 si la coupole 3 est à une bonne ou une mauvaise position par rapport au champ opératoire 2, plus précisément à indiquer à l'opérateur 6 si la coupole 3 est à une certaine distance prédéterminée du champ opératoire 2, correspondant à la distance optimale d'éclairage où les performances optiques de la coupole 3 sont les meilleures.

La distance prédéterminée est par exemple une distance de un mètre entre la coupole 3 et le champ opératoire 2.

Pour aider visuellement l'opérateur 6 à orienter et positionner manuellement la coupole 3 par rapport au champ opératoire 2 à la distance prédéterminée, le système de réglage comprend au moins une première source de lumière pour générer un premier faisceau de lumière en direction du champ opératoire 2 en formant une première tache d'éclairement sur le champ opératoire, et une seconde source de lumière pour générer un second faisceau de lumière en direction du champ opératoire 2 en formant une seconde tache d'éclairement sur le champ opératoire 2, les première et seconde sources de lumière étant disposées pour que les premier et second faisceaux convergent en un point 8 sur le champ opératoire 2 quand la distance prédéterminée est atteinte et qu'en dehors de cette distance, c'est-à-dire lorsque la coupole 3 est soit trop approchée soit trop éloignée du champ opératoire 2, les premiers et second faisceaux forment deux taches d'éclairement qui sont disjointes.

Sur la figure 2 est ici illustré l'agencement de trois sources de lumière, générant trois faisceaux de lumière en direction du champ opératoire 2. Les sources sont disposées sur la coupole 3 en décalage angulaire, et sont ici symétriquement réparties sur la périphérie de la coupole 3.

Comme cela est aussi illustré sur la figure 2, si les trois faisceaux de lumière générés convergent sur le même point 8 de sorte que les taches d'éclairement sont confondues sur le champ opératoire 2, alors l'opérateur 6 sait que la coupole 3 est correctement positionnée, c'est-à-dire que la distance prédéterminée est bien atteinte. Si la coupole 3 est trop proche, ou trop éloignée, alors les faisceaux de lumière générés ne convergent pas sur le même point 8 et les faisceaux de lumière forment trois taches d'éclairement qui sont disjointes sur le champ opératoire 2 (illustré par des lignes en pointillées). L'opérateur 6 comprend alors qu'il doit modifier le positionnement de la coupole 3 de sorte à atteindre la distance prédéterminée entre la coupole 3 d'éclairage et le champ opératoire 2 pour bénéficier de meilleures performances optiques de la coupole 3.

Selon l'invention, il est avantageux de multiplier les sources de lumière du système d'aide au réglage de la coupole 3 car dans le cas où il y ait au moins trois sources de lumière, le système d'aide au réglage restera fonctionnel même si l'opérateur 6 cache un des faisceaux de lumière en saisissant la coupole 3 par la poignée 7 par exemple pour la déplacer.

Selon un premier mode de réalisation, chaque faisceau de lumière projette sur le champ opératoire 2 une tache d'éclairement en forme de cercle plein, comme illustré sur les figures 3A et 3B.

Les figures 3A et 3B illustrent respectivement un mauvais positionnement de la coupole 3 par rapport au champ opératoire 2, la distance prédéterminée étant non atteinte, et un bon positionnement de la coupole 3 par rapport au champ opératoire 2, la distance prédéterminée étant atteinte. Sur la figure 3A, trois taches d'éclairement sous forme de cercle plein sont distinctement disjointes sur le champ opératoire 2. L'opérateur 6 doit alors ajuster le positionnement de la coupole 3 de sorte à obtenir l'image de la figure 3B sur laquelle les trois faisceaux convergent sur un même point, les taches d'éclairement sous forme de cercle plein étant confondues, informant visuellement l'opérateur 6 que la distance prédéterminée entre la coupole 3 et le champ opératoire 2 est atteinte et que la coupole 3 est correctement positionnée.

Selon un second mode de réalisation non représenté, chaque faisceau de lumière projette sur le champ opératoire 2 une tache d'éclairement en forme de cercle évidé.

Selon un troisième mode de réalisation, chaque faisceau de lumière projette sur le champ opératoire 2 une tache d'éclairement en forme de pointe de flèche, comme illustré sur les figures 4A, 4B et 4C.

Les figures 4A, 4B, et 4C illustrent respectivement un positionnement de la coupole 3 trop éloigné du champ opératoire 2, un positionnement de la coupole 3 à la distance prédéterminée par rapport au champ opératoire 2 et un positionnement de la coupole 3 trop proche du champ opératoire 2.

Sur la figure 4A, les pointes de flèche sont orientées vers un même point du champ opératoire 2 mais ne convergent pas sur ce point et sont visuellement disjointes. Cette configuration indique visuellement à l'opérateur 6 que la coupole 3 est trop éloignée du champ opératoire 2 et que pour atteindre la distance prédéterminée, l'opérateur 6 doit alors approcher manuellement la coupole 3 du champ opératoire 2 de sorte à obtenir la configuration visible sur la figure 4B dans laquelle les pointes de flèche convergent sur un même point et se touchent. Si l'opérateur 6 au cours du déplacement de la coupole 3 approche la coupole 3 du champ opératoire 2 au delà de la distance prédéterminée, alors la configuration de la figure 4C sera visible sur le champ opératoire 2, avec les trois pointes de flèche qui sont séparées, avec une orientation simulant un éloignement des pointes de flèche les unes des autres.

Selon l'invention, toutes les formes de tache d'éclairement projetées sur le champ opératoire 2 par les sources de lumière sont utilisables.

Selon l'invention, les sources de lumière du système d'aide au réglage peuvent être par exemple des sources de lumière laser, des LEDs, mais peuvent aussi être des sources de lumière choisies parmi celles agencées dans des modules d'éclairage de la coupole 3 d'éclairage. Au cours du déplacement de la coupole 3 d'éclairage par rapport au champ opératoire 2, elles peuvent être éclairées en continue ou en clignotant, à une fréquence de 5 hertz par exemple, avec une possibilité d'avoir un clignotement alternatif selon comment l'opérateur 6 estime que la convergence des faisceaux est la plus facile à apprécier visuellement.

Selon l'invention, la génération des faisceaux de lumière du système d'aide au réglage peut être activée manuellement par l'opérateur 6, lorsque celui touche par exemple un capteur capacitif agencé sur la poignée 7. Elle peut aussi être activée automatiquement à partir du moment où le système d'aide au réglage détecte que la coupole 3 d'éclairage est déplacée.

Selon l'invention, le système d'aide au réglage générant les faisceaux lumineux projetant les taches d'éclairement indique aussi à l'opérateur 6 dans quelle zone vise une caméra si celle-ci est par exemple agencée dans la poignée 7.

## Revendications

1. Dispositif d'éclairage (1) médical d'un champ opératoire (2) comprenant une coupole (3) d'éclairage à éclairage axial qui est destinée à être montée déplaçable au-dessus dudit champ opératoire (2) de façon à pouvoir être approchée ou éloignée manuellement dudit champ opératoire (2), et un système d'aide au réglage d'une bonne position d'éclairage de ladite coupole (3) par rapport audit champ opératoire (2) de telle manière à positionner ladite coupole (3) à une distance prédéterminée dudit champ opératoire (2), **caractérisé en ce que** ledit système d'aide au réglage comprend au moins une première source de lumière pour générer un premier faisceau de lumière et une seconde source de lumière pour générer un second faisceau de lumière, **en ce que** lesdites sources de lumière sont disposées sur ladite coupole (3) en décalage angulaire de telle manière que lesdits premier et second faisceaux de lumière convergent en un point (8) sur ledit champ opératoire (2) quand ladite distance prédéterminée est atteinte et qu'en dehors de cette distance lesdits deux faisceaux de lumière forment deux taches d'éclairement qui sont disjointes.

2. Dispositif selon la revendication 1, **caractérisé en ce qu'**il comprend en outre une troisième source de lumière pour générer un troisième faisceau de lumière, **en ce que** lesdites première, seconde et troisième sources de lumière sont disposées sur ladite coupole (3) en décalage angulaire de telle manière que lesdits premier, second et troisième faisceaux de lumière convergent audit point (8) sur ledit champ opératoire (2) quand ladite distance prédéterminée est atteinte et qu'en dehors de ladite distance lesdits trois faisceaux de lumière forment trois taches d'éclairement sur ledit champ opératoire (2) qui sont disjointes.

3. Dispositif selon l'une des revendications précédentes, **caractérisé en ce que** chaque faisceau de lumière projette sur ledit champ opératoire (2) une tache d'éclairement en forme de cercle plein, et de sorte que lesdits cercles pleins sont confondus sur ledit point (8) lorsque ladite coupole (3) est à ladite distance prédéterminée dudit champ opératoire (2).

4. Dispositif selon l'une des revendications 1 ou 2, **caractérisé en ce que** chaque faisceau de lumière projette sur ledit champ opératoire (2) une tache d'éclairement en forme de cercle évidé, et de sorte que lesdits cercles évidés sont confondus sur ledit point (8) lorsque ladite coupole (3) est à ladite distance prédéterminée dudit champ opératoire (2).

5. Dispositif selon l'une des revendications 1 ou 2, **caractérisé en ce que** chaque faisceau de lumière projette sur ledit champ opératoire (2) une tache d'éclairement en forme de pointe de flèche, de sorte que lesdites pointes de flèche sont orientées vers ledit point (8) lorsque ladite coupole (3) est à une distance supérieure à ladite distance prédéterminée dudit champ opératoire (2), de sorte que lesdites pointes de flèche converge vers ledit point (8) lorsque ladite coupole (3) est à ladite distance prédéterminée dudit champ opératoire (2) et de sorte que lesdites pointes de flèches s'éloignent dudit point (8) lorsque ladite coupole (3) est à une distance inférieure à ladite distance prédéterminée dudit champ opératoire (2).

6. Dispositif selon l'une des revendications précédentes, **caractérisé en ce que** lesdites sources de lumière dudit système d'aide au réglage sont des sources de lumière laser.

7. Dispositif selon l'une des revendications 1 à 5, **caractérisé en ce que** lesdites sources de lumière dudit système d'aide au réglage sont des LEDs.

## Patentansprüche

1. Medizinische Beleuchtungsvorrichtung (1) für ein Operationsfeld (2), umfassend eine Beleuchtungskuppel (3) mit axialer Beleuchtung, die dazu bestimmt ist, verlagerbar oberhalb des Operationsfeldes (2) derart montiert zu werden, dass sie manuell bezüglich des Operationsfeldes (2) angenähert oder entfernt werden kann, und ein Hilfssystem für die Einstellung einer guten Beleuchtungsposition der Kuppel (3) in Bezug auf das Operationsfeld (2) derart, dass die Kuppel (3) in einem vorbestimmten Abstand zu dem Operationsfeld (2) positioniert wird, **dadurch gekennzeichnet,**
**dass** das Hilfssystem für die Einstellung wenigstens eine erste Lichtquelle zum Erzeugen eines ersten Lichtstrahls und eine zweite Lichtquelle zum Erzeugen eines zweiten Lichtstrahls umfasst,
**dass** die Lichtquellen auf der Kuppel (3) im Winkelversatz derart angeordnet sind, dass die ersten und zweiten Lichtstrahlen in einem Punkt (8) auf dem Operationsfeld (2) konvergieren, wenn der vorbestimmte Abstand erreicht ist und dass außerhalb dieses Abstandes die beiden Lichtquellen (2) getrennte Beleuchtungsflecken erzeugen.

2. Vorrichtung nach Anspruch 1, **dadurch gekennzeichnet, dass** sie ferner eine dritte Lichtquelle zum Erzeugen eines dritten Lichtstrahls umfasst, dass die ersten, zweiten und dritten Lichtquellen auf der Kuppel (3) im Winkelversatz derart angeordnet sind, dass die ersten, zweiten und dritten Lichtquellen an dem Punkt (8) auf dem Operationsfeld (2) konvergieren, wenn der vorbestimmte Abstand erreicht ist, und dass außerhalb dieses Abstandes die drei Lichtquellen drei getrennte Beleuchtungsflecken auf dem Operationsfeld (2) erzeugen.

3. Vorrichtung nach einem der vorhergehenden Ansprüche, **dadurch gekennzeichnet, dass** jeder Lichtstrahl auf das Operationsfeld (2) einen Beleuchtungsfleck in Gestalt eines gefüllten Kreises projiziert, derart, dass die gefüllten Kreise auf dem Punkt (8) zusammenfallen, wenn die Kuppel (3) in dem vorbestimmten Abstand zu dem Operationsfeld (2) ist.

4. Vorrichtung nach einem der Ansprüche 1 oder 2, **dadurch gekennzeichnet, dass** jeder Lichtstrahl auf das Operationsfeld (2) einen Beleuchtungsfleck in Gestalt eines leeren Kreises projiziert, derart, dass die leeren Kreise auf dem Punkt (8) zusammenfallen, wenn die Kuppel (3) in dem vorbestimmten Abstand zu dem Operationsfeld (2) ist.

5. Vorrichtung nach einem der Ansprüche 1 oder 2, **dadurch gekennzeichnet, dass** jeder Lichtstrahl auf das Operationsfeld (2) einen Beleuchtungsfleck in Gestalt einer Pfeilspitze projiziert, derart, dass die Pfeilspitzen zu dem Punkt (8) orientiert sind, wenn die Kuppel (3) in einem größeren Abstand zu den vorbestimmten Abstand vom Operationsfeld (2) sind, derart, dass die Pfeilspitzen zu dem Punkt (8) konvergieren, wenn die Kuppel (3) in dem vorbestimmten Abstand zu dem Operationsfeld (2) ist und derart, dass die Pfeilspitzen sich von dem Punkt (8) entfernen, wenn die Kuppel (3) in einem kleineren Abstand als der vorbestimmte Abstand zu dem Operationsfeld (2) ist.

6. Vorrichtung nach einem der vorhergehenden Ansprüche, **dadurch gekennzeichnet, dass** die Lichtquellen des Hilfssystem für die Einstellung Laser-Lichtquellen sind.

7. Vorrichtung nach einem der Ansprüche 1 bis 5, **dadurch gekennzeichnet, dass** die Lichtquellen des Hilfssystem für die Einstellung LEDs sind.

## Claims

1. A medical lighting device (1) for illuminating an operating area (2), the device comprising an overhead light (3) providing axial illumination and designed to be movably mounted over said operating area (2) so as to be capable of being moved manually towards or away from said operating area (2), and a system for assisting adjustment of a proper lighting position for said overhead light (3) relative to said operating area (2) in such a manner as to position said overhead light (3) at a predetermined distance from said operating area (2), the device being **characterized in that** said system for assisting adjustment comprises at least a first light source for generating a first light beam and a second light source for generating a second light beam, **in that** said light sources are arranged on said overhead light (3) in angularly offset positions so that said first and second light beams converge at a point (8) on said operating area (2) when said predetermined distance is reached, and otherwise when not at said distance said two light beams form two light spots that are disjoint.

2. A device according to claim 1, **characterized in that** it further comprises a third light source for generating a third light beam, **in that** the first, second, and third light sources are arranged on said overhead light (3) in angularly offset positions in such a manner that the first, second, and third light sources converge at said point (8) on said operating area (2) when said predetermined distance is reached, and otherwise when not at said distance said three light beams form three light spots on said operating area (2) that are disjoint.

3. A device according to either preceding claim, **characterized in that** each light beam projects a light spot on said operating area (2) in the form of a solid circle, and in such a manner that said solid circles coincide at said point (8) when said overhead light (3) is at said predetermined distance from said operating area (2).

4. A device according to claim 1 or claim 2, **characterized in that** each light beam projects a light spot on said operating area (2) in the form of a hollow circle, and in such a manner that said hollow circles coincide at said point (8) when said overhead light (3) is at said predetermined distance from said operating area (2).

5. A device according to claim 1 or claim 2, **characterized in that** each light beam projects a light spot on said operating area (2) in the form of an arrowhead in such a manner that said arrowheads point towards said point (8) when said overhead light (3) is at a distance greater than said predetermined distance from said operating area (2), in such a manner that said arrowheads converge on said point (8) when said overhead light (3) is at said predetermined distance from said operating area (2), and in such a manner that said arrowheads point away from said point (8) when said overhead light (3) is at a distance less than said predetermined distance from said operating area (2).

6. A device according to any preceding claim, **characterized in that** said light sources of said system for assisting adjustment are laser light sources.

7. A device according to any one of claims 1 to 5, **characterized in that** said light sources of said system for assisting adjustment are LEDs.
